# EUROPEAN PATENT SPECIFICATION

(11) **EP 4 611 829 B1**
(45) Date of publication and mention of the grant of the patent: **23.09.2026**
(21) Application number: 23798492.7
(22) Date of filing: 06.11.2023
(51) Int. Cl.: A61L 27/38, A61L 27/60, C12N 5/071

(54) **SKIN SUBSTITUTES COMPRISING HUMAN SKIN BLOOD AND LYMPHATIC CAPILLARIES FROM 3D IN-VITRO SYSTEMS**
HAUTERSATZSTOFFE MIT MENSCHLICHEM HAUTBLUT UND LYMPHATISCHEN KAPILLAREN AUS 3D-IN-VITRO-SYSTEMEN
SUBSTITUTS DE PEAU COMPRENANT DU SANG HUMAIN DE LA PEAU ET DES CAPILLAIRES LYMPHATIQUES DE SYSTÈMES IN VITRO 3D

(30) Priority: 04.11.2022 EP 22205605
(43) Date of publication of application: 10.09.2025
(73) Proprietor: Universität Zürich, 8006 Zürich (CH)
(72) Inventor: BIEDERMANN, Thomas, 8006 Zürich (CH); KLAR, Agnes Silvia, 8006 Zürich (CH); RÜTSCHE, Dominic, 8006 Zürich (CH); PONTIGGIA, Luca, 8006 Zürich (CH); MÖHRLEN, Ueli, 8006 Zürich (CH)
(74) Representative: Schulz Junghans Patentanwälte PartGmbB
(86) International application number: PCT/EP2023/080757
(87) International publication number: WO 2024/094887

(56) References cited:
- EP-B1- 3 174 563
- MARINO DANIELA ET AL: "Bioengineering Dermo-Epidermal Skin Grafts with Blood and Lymphatic Capillaries", vol. 6, no. 221, 29 January 2014 (2014-01-29), XP093039289, ISSN: 1946-6234, Retrieved from the Internet <URL:https://www.science.org/doi/pdf/10.1126/scitranslmed.3006894> DOI: 10.1126/scitranslmed.3006894
- BOURLAND JENNIFER ET AL: "Tissue-engineered 3D melanoma model with blood and lymphatic capillaries for drug development", vol. 8, no. 1, 4 September 2018 (2018-09-04), pages 13191, XP055943966, Retrieved from the Internet <URL:https://www.nature.com/articles/s41598-018-31502-6.pdf> DOI: 10.1038/s41598-018-31502-6

## Description

This application claims the right of priority of European Patent Application EP22205605.3 filed 04 November 2022.

### Field

The present invention relates to the production of skin substitutes with a defined ratio of blood endothelial cells (BEC), to lymphatic endothelial cells (LEC), and their use in medicine.

### Background

Cells and tissues are inherently dependent on the vasculature to supply critical nutrients and oxygen in exchange for metabolites. Therefore, concerning skin, impaired wound vascularization can impede healing, resulting e.g., in scar-like dermal structures and tissue shrinkage. Several approaches including delivery of proangiogenic growth factors and cell-based therapies have been employed to enhance vascularization rate.

The latter strategy, the so called prevascularization approach, offers an innovative and effective therapeutic option to enhance wound healing. For this, microvascular capillary networks with dimensions and features resembling the human dermal microvasculature are being incorporated in biocompatible biomaterials such as collagen type I, fibrin, PEG, silk scaffolds and many others. Those biomaterials are seeded with human endothelial cells (EC). To generate prevascularized dermal substitutes endothelial cells are co-seeded with dermal fibroblasts. Subsequently, epidermal keratinocyte are seeded on top of the prevascularized dermal substitutes to engineer prevascularized dermo-epidermal skin substitute. After transplantation, capillaries embedded in vascularized skin substitutes rapidly connect (anastomose) with host's blood and lymphatic vasculature, respectively.

This connection process, called inosculation or anastomosis, is characterized by a close interaction of the preformed capillaries and the host's vasculature of the wound bed. It can take place either as internal or external inosculation. The first occurs within an implant and involves regression of the tissue-engineered graft's capillaries with subsequent replacement by invading host microvessels. In contrast, external inosculation is characterized by the outgrowth of graft's capillaries and reconnection to the microvasculature of the wound bed. Importantly, inosculation allows reperfusion of transplanted tissues within 4 days and is advantageous when compared with the non-prevascularized tissue-engineered grafts, which are initially entirely dependent on diffusion, which is a slow and inefficient process leading to inadequate delivery of oxygen, nutrients, and growth factors. Consequently, the pre-vascularization has been shown to promote cell survival, differentiation, and physiological integration of the engineered tissue.

Marino et al. ((2014) Science Translational Medicine, vol. 6, no. 221) discloses a method wherein HDMECs and fibroblasts are isolated from foreskin by scratching and cocultured to obtain human skin grafts. Bourland et al. ((2018) Scientific Reports, vol. 8, no. 1, p. 13191) discloses the production of a 3D melanoma model system. HDMECs and fibroblasts are isolated together via scratching from resected skin of healthy donors. EP 3 174 563 B1 discloses the production of skin grafts from foreskin or adipose tissue samples. HDMECs and fibroblasts are isolated together and cultured on gelatin-coated dishes. Fibroblasts are removed by scratching.

Based on the above-mentioned state of the art, the objective of the present invention is to provide means and methods to treat certain skin diseases and defects of the skin using a skin substitute. This objective is attained by the subject-matter of the independent claims of the present specification, with further advantageous embodiments described in the dependent claims, examples, figures and general description of this specification.

### Summary of the Invention

With regards to the primary cell isolation from a single skin biopsy, the inventors established a method to isolate 5 distinct cell populations at the same time: keratinocytes and melanocytes from the epidermis (upper skin part) and fibroblasts, pericytes and endothelial cells from the dermis (lower part). Most importantly, the inventors can separate blood and lymphatic endothelial cells.

Fibroblasts and EC are isolated from the dermis by collagenase type I digestion followed by EC enrichment by magnetic sorting. Subsequently, BEC and LEC fractions of the EC are separated by flow cytometry or magnetic sorting after a short in vitro cultivation time. Whereas BEC represent CD31 positive Podoplanin negative fraction of all EC, LEC are CD31 positive Podoplanin positive cells. Further, BEC can be separated in arterial (Neuropilin 1 positive, Ephrin B positive) and venous (Neuropilin 2, ACKR1 (DARC) positive) cells.

Concomitantly, pericytes can be enriched from the dermal cell fraction by magnetic sorting for CD146 marker. Pericytes or perivascular/mural cells covering microvascular capillaries in skin play an essential role in maintaining vascular stability and vascular remodeling.

The inventors further describe the use of the isolated and cultured cells to prepare a prevascularized skin substitute with various ratios of BEC to LEC that can include also pericytes, fibroblasts, melanocytes and keratinocytes. The different ratios of BEC to LEC result in prevascularized skin substitutes that are intended to support skin wound healing for different skin disorders.

In particular, the skin substitutes containing 100%/0% BEC/LEC can be applied for indications such as ischemic (arterial) ulcers, venous leg ulcers, or acute burns.

Skin substitutes containing 0/100% BEC/LEC can be applied for indications such as secondary lymphodema, lipedema, elephantiasis (lymphatic filariasis), or lymphangioleiomyomatosis.

Skin substitutes containing a mixed ratio of BEC/LEC (50/50%) can be applied for e.g. phlebolymphedema and reconstructive skin surgery.

A first aspect of the invention relates to a method for production of a skin substitute, said method comprising the steps:
a. providing a skin tissue sample isolated from a mammalian subject;
b. separating the skin tissue sample into dermis and epidermis;
c. enzymatically degrading matrix components of the dermis to obtain a plurality of cells in suspension;
d. in a CD31 step, sorting said plurality of cells into a CD31⁺ cell fraction and a CD31-cell fraction;
e. culturing the CD31⁺ cell fraction in appropriate culture medium under appropriate cell culture conditions yielding a plurality of CD31⁺ cells;
f. in a BEC/LEC step, sorting said plurality of CD31⁺ cells into blood endothelial cells (BEC), and lymphatic endothelial cells (LEC) using a BEC-specific and/or LEC-specific marker;
g. in a culturing step, culturing BEC and/or LEC in appropriate culture medium under appropriate cell culture conditions yielding a skin substitute.

A second aspect of the invention relates to a skin substitute obtained by the method according to the first aspect.

Further aspects of the invention relate to a skin substitute according to the second aspect for use in treatment of certain skin diseases.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

The term "if transplanted" refers to the condition of a skin substitute as disclosed herein under conditions post transplantation, and may be read alternatively as "when transplanted" also.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

### Cell Biology, diagnostic method inventions: Markers, ligands

In the present specification, the term *positive,* when used in the context of expression of a marker, refers to expression of an antigen assayed by a fluorescently labelled antibody, wherein the label's fluorescence on the structure (for example, a cell) referred to as "positive" is at least 30% higher (≥ 30 %), particularly ≥50% or ≥80%, in median fluorescence intensity in comparison to staining with an isotype-matched fluorescently labelled antibody which does not specifically bind to the same target. Such expression of a marker is indicated by a superscript "plus" (⁺), following the name of the marker, e.g. CD31⁺, or by the addition of the plus sign after the marker name ("CD31⁺"). If the word "expression" is used herein in the context of "gene expression" or "expression of a marker or biomolecule" and no further qualification of "expression" is mentioned, this implies "positive expression" as defined above.

In the present specification, the term *negative,* when used in the context of expression of a marker, refers to expression of an antigen assayed by a fluorescently labelled antibody, wherein the median fluorescence intensity is less than 30% higher, particularly less than 15% higher, than the median fluorescence intensity of an isotype-matched antibody which does not specifically bind the same target. Such expression of a marker is indicated by a superscript minus (⁻), following the name of the marker, e.g. CD31⁻, or by the addition of the minus sign after the marker name ("CD31⁻").

*High* expression of a marker, for example high expression of NRP1, refers to the expression level of such marker in a clearly distinguishable cell population that is detected by FACS showing the highest fluorescence intensity per cell compared to the other populations characterized by a lower fluorescence intensity per cell. A high expression is indicated by superscript "high" or "hi" following the name of the marker, e.g. NRP1^{high}. The term "is expressed highly" refers to the same feature.

Low expression of a marker, for example low expression of NRP1, refers to the expression level of such marker in a clearly distinguishable cell population that is detected by FACS showing the lowest fluorescence intensity per cell compared to the other populations characterized by higher fluorescence intensity per cell. A low expression is indicated by superscript "low" or "lo" following the name of the marker, e.g. NRP1^{low}. The term "is expressed lowly" refers to the same feature.

The expression of a marker may be assayed via techniques such as fluorescence microscopy, flow cytometry, ELISPOT, ELISA or multiplex analyses.

The term *skin substitute* in the context of the present specification relates to a 3D scaffold of skin tissue being produced in the laboratory according to the invention. The skin substitute is an *ex vivo* grown skin tissue. The term skin mimetic is equivalent to the term skin substitute. The skin substitute can be autologous or allogenic.

The term *skin tissue sample* in the context of the present specification relates to samples comprising, as a minimum, epidermis and dermis. These two tissue types are sufficient to isolate all 5 cell types mentioned below. Size requirements for the sample are at minimum a 4 mm (punch) biopsy ((4 mm in diameter punch biopsy needles are commonly used in dermatology to excise skin pieces for histopathological examinations)) or larger.

The term *dermis* or *dermal part of the substitute* in the context of the present specification relates to a layer of skin between the epidermis and subcutaneous tissues. Structural components of the dermis are collagen, elastic fibers, and extrafibrillar matrix. Nerves and lymphatic and blood vessels are present in the dermis.

The term *epidermis* or *epidermal part of the substitute* in the context of the present specification relates to the outermost of the three layers that comprise the skin. The epidermis primarily consists of keratinocytes, but may also contain melanocytes.

The term *relative amount of BEC to LEC* in the context of the present specification relates to a pre-determined ratio between BEC and LEC. The determination of the ratio is done depending on the purpose of the skin substitute. Each indication for applying a skin substitute according to the invention requires a defined ratio of BEC to LEC ranging from 100% BEC and 0% LEC to 0% BEC and 100% LEC.

The term FACS in the context of the present specification relates to fluorescence-activated cell sorting.

As used herein, the term *treating* or *treatment* of any disease or disorder (e.g. a skin disease) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described herein below.

### Detailed Description of the Invention

A first aspect of the invention relates to a method for production of a skin substitute. The method comprises the steps:
In step a, a skin tissue sample is provided isolated from a mammalian subject. The isolated sample is supplemented with antibiotics, and fungicides.

In step b, the skin tissue sample is separated into dermis and epidermis. In certain embodiments, the separation is achieved using forceps. In certain embodiments, the separation is achieved using a microscope. In certain embodiments, the separation is achieved without the use of an optical device.

In step c, matrix components of the dermis are enzymatically degraded to obtain a plurality of cells in suspension. This step may take ~20 min to ~2 hours (depending on the size of the sample). It is necessary to destroy the so-called extracellular matrix in which the endothelial cells are located and to use a digestion to destroy the connection of the cells to each other to yield single cells that then the cells can be sorted.

In step d, also referred to as the *CD31 step* herein, the plurality of cells is sorted into a CD31⁺ cell fraction and a CD31⁻ cell fraction. This can be achieved by common methods of cell separation that discern cells by their expression, or absence thereof, of cell surface markers, in this context, CD31.

In step e, the CD31⁺ cell fraction is cultured in appropriate culture medium under appropriate cell culture conditions yielding a plurality of CD31⁺ cells.

In step f, also called BEC/LEC step, the plurality of CD31⁺ cells is sorted into blood endothelial cells (BEC), and lymphatic endothelial cells (LEC) using a BEC-specific and/or LEC-specific marker.

In step g, also called culturing step, a plurality of cells comprising BEC and/or LEC is cultured in appropriate culture medium under appropriate cell culture conditions yielding a skin substitute.

Appropriate cell culture conditions comprise a temperature of ~37°C, CO₂ concentration of ~5%, and a relative humidity of ~95%. In certain embodiments, the cells are cultivated in endothelial cell medium.

In certain embodiments, in the culturing step, a relative amount of BEC to LEC is used between 100% BEC and 0% LEC to 0% BEC and 100% LEC. In certain embodiments, the plurality of cells comprising BEC and/or LEC of the culturing step consists of 100% BEC. In certain embodiments, the plurality of cells comprising BEC and/or LEC of the culturing step consists of 100% LEC. In certain embodiments, the plurality of cells comprising BEC and/or LEC of the culturing step comprises BEC and certain other cells mentioned below, but no LEC. In certain embodiments, the plurality of cells comprising BEC and/or LEC of the culturing step comprises LEC and certain other cells mentioned below, but no BEC.

In certain embodiments, in the culturing step, a relative amount of BEC to LEC is used between 70% BEC and 30% LEC to 30% BEC and 70% LEC.

In certain embodiments, a relative amount of BEC to LEC is chosen depending on the purpose of the skin substitute. This means that each indication which is treated with the skin substitute of the invention has its unique challenges and requires an adapted amount of BEC and/or LEC.

In certain embodiments, the CD31 step is performed via magnetic sorting.

In certain embodiments, the CD31 step is performed via paramagnetic particles. In certain embodiments, the paramagnetic particles are uniform polystyrene spherical beads that have been made magnetically susceptible. The paramagnetic particles enable gentle binding of low-abundance antigens or rare cell populations. The method exhibits minimal shear force exerted on the cells when compared to MACS and FACS.

In certain embodiments, the paramagnetic particles are 1 to 5 micrometers in diameter. In general, as a first step, the use of paramagnetic particles is most gentle for the cell (survival of the cells) when being isolated from a skin tissue.

In certain embodiments, the BEC/LEC step is performed via fluorescence-based sorting or magnetic sorting. In certain embodiments, the BEC/LEC step is performed via FACS.

In certain embodiments, in the BEC/LEC step, the BEC-specific marker is selected from the group consisting of PLVAP, and PAL-E.

In certain embodiments, in the BEC/LEC step, the LEC-specific marker is selected from the group consisting of Podoplanin, Lyve1, and VEGFR3.

In certain embodiments, the skin tissue sample is derived from non-fetal tissue. In certain embodiments, the non-fetal tissue is derived from the same patient who receives the skin substitute. The advantage of using tissue derived from the same patient who receives the skin substitute is that the skin substitute will not cause an immune rejection reaction.

In certain embodiments, the non-fetal tissue is derived from surgical removal of the foreskin. In certain embodiments, the skin tissue sample is derived from fetal tissue. In certain embodiments, the fetal skin tissue is derived from a spina bifida surgery. The advantage of using tissue derived from a tissue donor is that the skin substitute is ready-to-use, while an autologously grown skin substitute would require ~3 weeks of preparation time. Also, the donor of the skin tissue is very young in most cases, and the fetal skin tissue or tissue of children has increased potential to differentiate into certain cell types, so that the resulting skin substitute integrates into the host skin more easily.

In certain embodiments, the skin tissue sample is derived from human tissue.

In certain embodiments, the skin tissue sample is derived from porcine tissue. Skin substitutes from porcine tissue have the advantage that more donor tissue is available, and the risk of transmitting infection from the skin donor to the recipient is reduced. In certain embodiments, the skin-donor pig is genetically modified so that its skin is less immunogenic and an immune rejection reaction of the recipient is less likely.

In certain embodiments, after the BEC/LEC step, BEC are sorted into arterial and venous endothelial cells via an arterial endothelial cell- and/or a venous endothelial cell-specific marker.

In certain embodiments, the arterial endothelial cell-specific marker is selected from the group consisting of Ephrin B2 positive, and Neuropilin 1 (NRP1)^{high}.

In certain embodiments, the venous endothelial cell-specific marker is selected from the group consisting of ACKR1 (DARC) positive, and Neuropilin 2 (NRP2)^{high}.

In certain embodiments, during the BEC/LEC step, additionally CD146⁺ mural cells (pericytes) are sorted (via their expression of CD146) from the CD31⁻ cell fraction and are added in the culturing step.

In certain embodiments, keratinocytes are isolated from the epidermis and keratinocytes are added in the culturing step.

In certain embodiments, the skin sample is first cut into small pieces and digested overnight at 4°C in ~12U/ml dispase. Thereafter, the epidermis is mechanically separated from the dermis using forceps.

In certain embodiments, keratinocytes are isolated via treatment of the epidermis with ~0.5% (w/v) trypsin solution. In certain embodiments, keratinocytes are isolated via sorting cells of the epidermis into a MC1R negative/E-cadherin positive fraction (which comprises the keratinocytes).

In certain embodiments, melanocytes are isolated from the epidermis and melanocytes are added in the culturing step.

In certain embodiments, melanocytes are isolated via treatment with ~0.05% (w/v) trypsin solution. In certain embodiments, melanocytes are isolated via sorting cells of the epidermis into a MC1R positive/E-cadherin positive fraction (which comprises the melanocytes).

Further, to isolate melanocytes/keratinocytes, the epidermis is digested to separate the cells from the so-called basement membrane and again also to separate the cells from each other.

In certain embodiments, digestion of the tissue sample is performed using an enzyme or an enzyme combination selected from the group consisting of collagenase II, and/or collagenase IV, and/or a mix of collagenase I and II, commercially available as "liberase", and/or trypsin.

In certain embodiments, in the culturing step, additionally
- collagen, and/or
- fibrin, and/or
- gelatin and/or
- PEG and/or
- polyurethane
is added. In certain embodiments, in the culturing step, additionally collagen is added. In certain embodiments, in the culturing step, additionally fibrin is added. In certain embodiments, in the culturing step, additionally gelatin is added. In certain embodiments, in the culturing step, additionally PEG is added. In certain embodiments, in the culturing step, additionally polyurethane is added.

In certain embodiments, to prepare a prevascularized hydrogel, collagen type I (bovine collagen type I) is mixed with a cell suspension of a 1:1 mixture of HDMEC and fibroblasts.

A second aspect of the invention relates to a skin substitute obtained by the method according to the first aspect. In certain embodiments, the skin substitute according to the second aspect comprises endothelial cells. In certain embodiments, the dermal part of the skin substitute according to the second aspect comprises ≥35% endothelial cells. In certain embodiments, the dermal part of the skin substitute according to the second aspect comprises ~50% endothelial cells. In certain embodiments, all cells of the skin substitute are derived from a single donor subject.

In certain embodiments, the skin substitute is an isolated skin substitute. This means that the skin substitute is grown *in vitro* and is distinguishable from a skin sample.

In certain embodiments, the skin substitute, if transplanted to a recipient subject, is capable of being connected to recipient subject's CD31⁺ blood capillaries 4 days after transplantation to the recipient subject. In certain embodiments, >15% of donor CD31⁺ blood capillaries of the skin substitute are capable of being connected to recipient subject's CD31⁺ blood capillaries 4 days after transplantation to the recipient subject. This feature is present in skin substitutes comprising BECs.

In certain embodiments, the skin substitute, if transplanted to a recipient subject, is capable of attracting ingrowth of recipient subject's blood capillaries throughout the dermis up to the basement membrane. In certain embodiments, the skin substitute, if transplanted to a recipient subject, is capable of attracting ingrowth of recipient subject's blood capillaries throughout the dermis up to the basement membrane at one week after transplantation to the recipient subject. This feature is present in all skin substitutes.

In certain embodiments, the skin substitute, if transplanted to a recipient subject, is capable of attracting ingrowth of recipient subject's blood capillaries at 4 days after transplantation to the recipient subject. This feature is present in all skin substitutes.

In certain embodiments, the skin substitute, if transplanted to a recipient subject, is capable of showing an oxygen saturation level of >50% at one week after transplantation to the recipient subject. This feature is present in skin substitutes comprising BECs.

In certain embodiments, the skin substitute, if transplanted to a recipient subject, is capable of having oxygenated hemoglobin levels of >30% at one week after transplantation to the recipient subject. This feature is present in skin substitutes comprising BECs.

In certain embodiments, the skin substitute, if transplanted to a recipient subject, is capable of comprising a higher number of M2 macrophages than number of M1 macrophages at three weeks after transplantation to the recipient subject. This feature is present in skin substitutes comprising BECs.

In certain embodiments, the skin substitute, if transplanted to a recipient subject, is characterized by low or absent expression of CK16 and CK17 (CK16^{low} and CK17^{low} or CK16⁻and CK17⁻) at two weeks after transplantation to the recipient subject, particularly at one week after transplantation to the recipient subject. This feature is present in skin substitutes comprising BECs.

In certain embodiments, a ratio of cell types in the dermis is selected from table 1, a ratio of arterial to venous BEC ratio is selected from table 2, and a ratio of cell types in the epidermis is selected from table 3. In certain embodiments, a ratio of cell types in the dermis is selected from table 1, and no epidermal cells are used.

The differences to existing skin substitutes are in the morphology, structure and function of blood and lymphatic capillaries. For example, blood capillaries show continuous basement membrane, pericytic coverage and express specific markers (Collagen IV) and are specialized to transport blood and participate in oxygen and nutrient exchange, whereas lymphatic capillaries do not show basement membrane, do not show pericytic coverage, express other specific markers (LYVE1, Podoplanin) and take up excessive fluid. The latter can be demonstrated with Evans blue assay.

Generally speaking, the skin substitute provided by the present invention can be employed in treatment of a skin defect caused by burn, or in reconstructive skin surgery. The invention is also useful in treatment of skin disease or chronic wounds.

A further aspect of the invention relates to a skin substitute according to the second aspect for use in treatment of a secondary lymphoedema. In certain embodiments, the skin substitute for use in treatment of a secondary lymphoedema was cultured from a relative BEC/LEC amount of 0% BEC and 100% LEC.

Secondary lymphedema is caused by damage to the lymphatic system or problems with the movement and drainage of fluid in the lymphatic system; it can be the result of a cancer treatment, an infection, injury, inflammation of the limb, or a lack of limb movement. Secondary lymphedema is caused by injury or obstruction of the lymphatic system, which occurs most frequently as a consequence of cancer treatment such as in breast cancer survivors, but is also a common complication of other solid tumors. In most cases, the development of lymphedema after surgery occurs in a delayed but permanent fashion, presenting months and sometimes even years after initial treatment. Therefore, skin substitutes with engineered lymphatic capillaries can take up the excessive interstitial fluid from the damaged (e.g., burned) area, thus reducing skin lymphedema.

A further aspect of the invention relates to a skin substitute according to the second aspect for use in treatment of a chronic edema. In certain embodiments, the skin substitute for use in treatment of a chronic edema was cultured from a relative BEC/LEC amount of ~50% BEC and ~50% LEC.

Chronic edema in the lower extremities is usually an expression of phlebolymphedema, a combination of venous and lymphatic dysfunction.

A further aspect of the invention relates to a skin substitute according to the second aspect for use in treatment of an acute burn wound. In certain embodiments, the skin substitute for use in treatment of an acute burn wound was entirely cultured from BEC cells, with no LEC (a relative BEC/LEC amount of 100% BEC and 0% LEC).

Burns are caused by injury to the skin from excessive heat or another injury. The heat can be the result of thermal, electrical, chemical, or electromagnetic energy. Ischemic necrosis develops within hours after burn injury, and is most prominent in directly involved burned tissues, but also develops in distant, uninjured tissue, including muscle, intestine and lung. Further, hematologic changes impair microvasculature and thus, blood perfusion of the burned tissue. Therefore, a skin substitute with engineered arterial/venous blood capillaries can provide oxygen and nutrients to the wound area, thus improving blood flow and healing of skin.

A further aspect of the invention relates to a skin substitute according to the second aspect for use in treatment of an arterial ulcer. In certain embodiments, the skin substitute for use in treatment of an arterial ulcer was cultured from a relative BEC/LEC amount of 100% BEC and 0% LEC. Therefore, a skin substitute with engineered arterial blood capillaries can provide oxygen and nutrients to the wound area, thus improving vascularization, healing and wound closure.

Arterial ulcers, also referred to as ischemic ulcers, are caused by poor perfusion/blood circulation in the arteries (delivery of nutrient-rich blood) to the lower extremities. Reduced circulation may be due to diabetes, inflammation, fat blockages, clogged arteries, peripheral arterial disease (PAD), or infection. When blood does not reach the extremities, the area fails to get adequate oxygen and nutrients. This causes tissue damage and even cell death. The damaged, blood deprived tissue is unable to heal. If untreated, ischemic ulcers can lead to gangrene and limb loss.

A further aspect of the invention relates to a skin substitute according to the second aspect for use in treatment of a venous leg ulcer. In certain embodiments, the skin substitute for use in treatment of a venous leg ulcer was cultured from a relative BEC/LEC amount of 100% BEC and 0% LEC.

Venous leg ulcer is the most common type of leg ulcer, accounting for more than 90% of all cases. Venous leg ulcers are chronic, long-lasting open sores, which usually develop on the inside of the leg, just above the ankle. Ulcers are caused by diseases of the leg veins such as varicose veins or as a complication of a DVT (Deep Vein Thrombosis). Venous ulcers typically occur because of damage to the valves inside the leg veins due to sustained venous hypertension causing ulcers formation on ankles. Therefore, a skin substitute with engineered venous blood capillaries, can provide better venous return of deoxygenated blood from the wound area, thus improving in general body blood flow.

A further aspect of the invention relates to a skin substitute according to the second aspect for use in treatment of lymphatic filariasis. In certain embodiments, the skin substitute for use in treatment of lymphatic filariasis was cultured from a relative BEC/LEC amount of 0% BEC and 100% LEC.

Elephantiasis (lymphatic filariasis) is a disease in which parasitic roundworms occupy and occlude lymphatic vessels leading to a painful and profoundly disfiguring disease. It is caused by infection with parasites classified as nematodes. In communities where filariasis is transmitted, all ages are affected. While the infection may be acquired during childhood its visible manifestations such as limbs edema may occur later in life, causing temporary or permanent disability. Therefore, a skin substitute with engineered lymphatic capillaries can take up the excessive interstitial fluid from limbs, thus reducing limbs edema.

A further aspect of the invention relates to a skin substitute according to the second aspect for use in treatment of phlebolymphedema. In certain embodiments, the skin substitute for use in treatment of phlebolymphedema was cultured from a relative BEC/LEC amount of ~50% BEC and ~50% LEC.

Phlebolymphedema is a mixed-etiology swelling due to chronic venous insufficiency and lymphatic insufficiency. Therefore, if one of the two systems should fail in its normal function (e.g., chronic venous hypertension, lymphedema), such mutual interdependence causes a new condition that affects both systems simultaneously known as phlebolymphedema, also called venolymphatic edema. Therefore, a skin substitute with engineered blood/lymphatic capillaries can provide oxygen and nutrients and at the same time take up the excessive interstitial fluid from limbs, thus improving blood flow and reducing tissue swelling.

A further aspect of the invention relates to a skin substitute according to the second aspect for use in treatment of lymphangioleiomyomatosis. In certain embodiments, the skin substitute for use in treatment of lymphangioleiomyomatosis was cultured from a relative BEC/LEC amount of 0% BEC and 100% LEC.

In patients with lymphangioleiomyomatosis, the lymphatic vessels may rupture or become blocked (obstructed) causing chyle to accumulate in the chest cavity (chylothorax). Chylothorax is a rare but serious condition in which lymph formed in the digestive system (chyle) accumulates in the chest cavity.

A further aspect of the invention relates to a skin substitute according to the second aspect for use in treatment of skin defect, wherein the skin substitute was cultured from a relative BEC/LEC amount as present in the defected skin. Such skin defect is (but not exclusively) for example, skin trauma after chemical burn, skin trauma after radiation, skin trauma after avulsion trauma. From a small patient skin biopsy the specific blood and lymphatic vasculature ratio is analyzed, then the skin substitutes will be prepared to include BEC and LEC in this patient specific ratio to apply the skin substitute to the wound.

**Table 1. Possible cell ratios in dermal part of skin substitutes**

| **Total cell ratio *100%*** | | | |
|---|---|---|---|
| **BEC/LEC ratio is total 50% of the cells** | | **Fibroblast and/or Pericyte ratio is total 50% of the cells** | |
| *BEC* | *LEC* | *Fibroblasts* | *Pericytes* |
| 25% | 25% | 50% | 0% |
| 35% | 15% | 50% | 0% |
| 15% | 35% | 50% | 0% |
| 0% | 50% | 50% | 0% |
| 25% | 25% | 30% | 20% |
| 35% | 15% | 30% | 20% |
| 15% | 35% | 40% | 10% |

**Table 2. Possible arterial to venous cell ratios in dermal part of BEC in skin substitutes**

| **BEC arterial to venous cells** | **Possible application (but not exclusive)** |
|---|---|
| 50% arterial to 50% venous | e.g. acute burn wound |
| 30%-0% arterial to 70%-100% venous | e.g. for venous leg ulcer |
| 100%-70% arterial to 0% - 30% venous | e.g. arterial ulcer |

**Table 3. Possible seeded cell ratios in epidermal part of skin substitutes**

| **keratinocytes** | **melanocytes** |
|---|---|
| 100% | 0% |
| 80% | 20% |

In the production process of the skin substitute, first the dermis is built by a cell culture of (a) BEC and/or LEC, (b) fibroblasts, and (c) possibly pericytes. After ~ one week of dermis culture, the epidermal part is added to the culture which consists of keratinocytes and possibly melanocytes. In certain embodiments, the dermis is sufficient to create a skin substitute, and no epidermal cells are added.

### Medical treatment

Similarly, within the scope of the present invention is a skin substitute for use in a method or treating a skin disease in a patient in need thereof, comprising administering to the patient a skin substitute according to the above description.

### Method of Manufacture and Method of Treatment according to the invention

The invention further encompasses, as an additional aspect, the use of a skin substitute as identified herein, for use in a method of manufacture of a medicament for the treatment or prevention of a skin disease.

Similarly, the invention encompasses a skin substitute for use in methods of treatment of a patient having been diagnosed with a skin disease associated with an open wound. This method entails administering to the patient a skin substitute as identified herein.

Wherever alternatives for single separable features such as, for example, a ratio or a marker protein, or an indication are laid out herein as "embodiments", it is to be understood that such alternatives may be combined freely to form discrete embodiments of the invention disclosed herein. Thus, any of the alternative embodiments for a ratio may be combined with any of the alternative embodiments of a marker protein and these combinations may be combined with any medical indication mentioned herein.

The invention is further illustrated by the following examples and figures, from which further embodiments and advantages can be drawn. These examples are meant to illustrate the invention but not to limit its scope.

### Description of the Figures

- Fig. 1: shows human CD31 (red) positive endothelial cell fraction after Dynabeads sorting on a cell culture plate. Cell nuclei are stained with Hoechst (blue). Scale bar: 50 µm.
- Fig. 2: shows human CD31 negative cell fraction after Dynabeads sorting on a cell culture plate. Cell nuclei are stained with Hoechst (blue). Scale bar: 50 µm.
- Fig. 3: shows FACS analysis of human BEC and LEC sorted cells (using Podoplanin as exemplary LEC specific marker). LEC co-express CD31 (endothelial specific marker) and Podoplanin, whereas BEC express CD31, but not Podoplanin.
- Fig. 4: shows human keratinocytes on a cell culture plate. Scale bar: 50 µm.
- Fig. 5: shows human melanocytes on a cell culture plate. Scale bar: 50 µm.
- Fig. 6: shows CD146 positive pericytes on a cell culture plate. The entire population of pericytes results positive for CD146, while fibroblasts (FBs) as control, displays no detection of CD146, respectively. CD146⁺ pericytes are also positive for NG2, while only a small percentage is also positive for Desmin. CD146⁺ pericytes are positive for αSMA and CD90 as also fibroblasts are positive for αSMA and CD90. Cell nuclei are stained with Hoechst (blue). Scale bar: 100 µm.
- Fig. 7: shows whole mount immunofluorescence staining for CD31 (red) and PROX1 (green) of a 3-dimensional scaffold containing sorted and cultured BEC (CD31⁺PROX1⁻) / LEC (CD31⁺PROX1⁺) and fibroblasts (not stained in this example) displaying capillary formation in vitro. Scale bar: 50 µm.

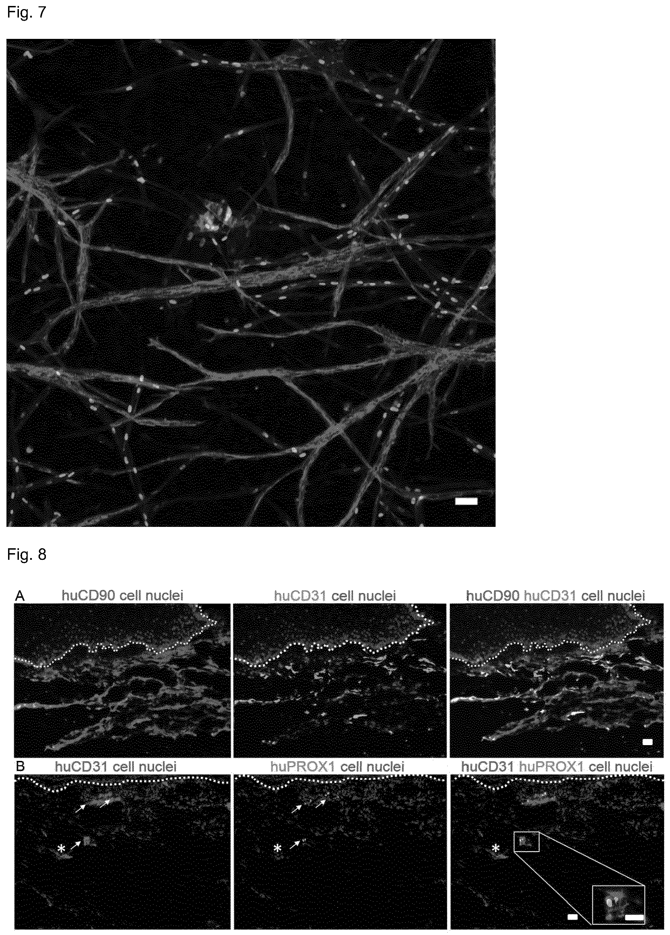

- Fig. 8: shows an immunofluorescence staining of a prevascularized human dermo-epidermal skin substitute 1 week after transplantation onto an immune-incompetent nude rat. (A) Immunofluorescence co-staining of a prevascularized skin substitute against human (hu) CD90 (red) and huCD31 (green). Shown are single stainings and an overlay. Human CD90 depicts specifically the human dermal compartment of the substitute, while CD31 is expressed exclusively on human endothelial cells. (B) Immunoflorescence co-staining of a skin substitute against huCD31 (red) as well as huPROX1 (green), visualizing the human blood (CD31⁺PROX1⁻) and lymphatic capillaries (CD31⁺PROX1⁺). White arrows indicate double-positive, lymphatic microvascular structures, whereas the star represents a single-positive blood capillary. An inset shows a magnification of a lymphatic capillary. The white dotted line indicates the dermal-epidermal junction. Cell nuclei are stained with Hoechst (blue). Scale bars 50 µm.
- Fig. 9: shows analysis of BEC-LEC seeded skin substitutes in vivo. (A) Establishment of a functional connection (anastomosis) between human and rat CD31⁻ positive capillaries after 4 days (4d), 1 (1w) and 2 (2w) weeks after transplantation *in vivo.* Mean ± SD, n = 5, *** *P*=0.0001 (d4), * *P*=0.0131 (1w), ns, *P*=0.2813 (2w), vs. non-vascularized control. (B) Quantification of rat capillary ingrowth (%) 4d after transplantation. Mean ± SD, n = 5, *** *P* <0.0001 (d4), vs. non-vascularized control.
- Fig. 10: shows animal model, photo-acoustic imaging. (A-B) BEC-LEC substitutes (white bars) and non-vascularized controls (black bars) were transplanted for 1 week (1w) into full-thickness skin defects on the nude rats. (A) B-mode ultrasound measurement. Quantification of oxygen saturation (%). Mean ± SD, n = 5, *** *P* =0.0059, vs. non-vascularized control. (B) Quantification of oxygenation level of hemoglobin (%). Mean ± SD, n = 5, **P* < 0.0372, vs. non-vascular control.

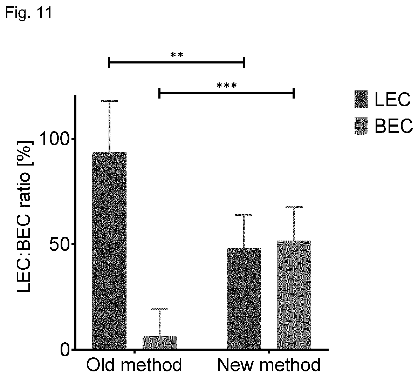

- Fig. 11: A comparison of LEC:BEC ratio in isolated endothelial cells (HDMEC) using two different isolation techniques. HDMEC were isolated from human foreskins using the newly established protocol of this application and compared to the standard procedure (EP 3 174 563 B1 and Marino et al. 2014). HDMEC isolations using the old scratching method (EP 3 174 563 B1 and Marino et al., 2014) revealed 93.13 ± 24.17% of LEC and 6.31 ± 13.09% of BEC (n= 48). In contrast, separation of HDMEC after isolation using the new isolation method (this application) yielded 47.92± 15.86% of LEC (P=0.0016) and 51.57± 16.09% of BEC (P=0.00048) (n= 35).

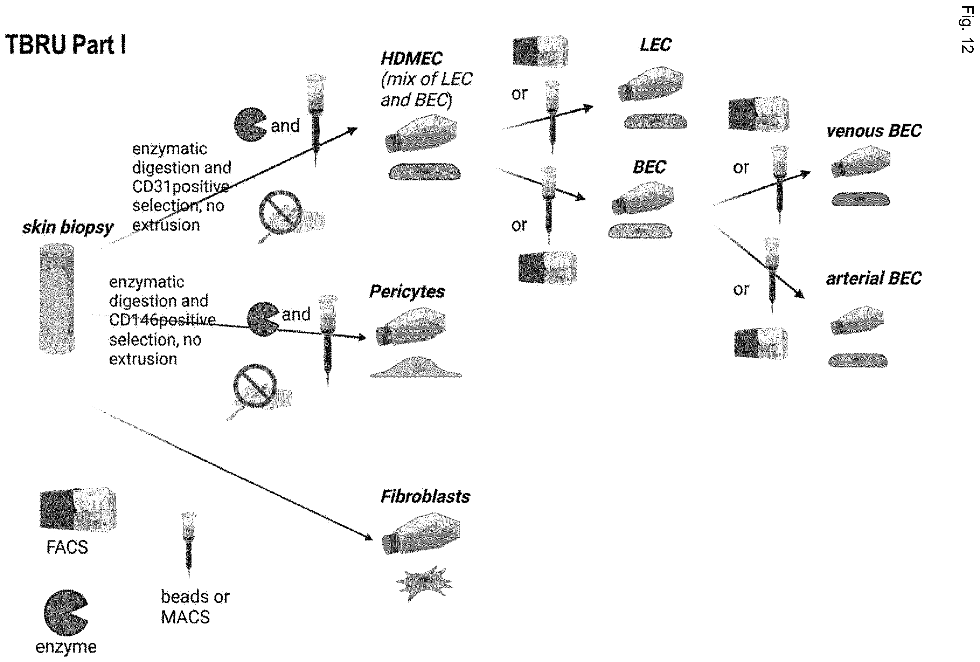

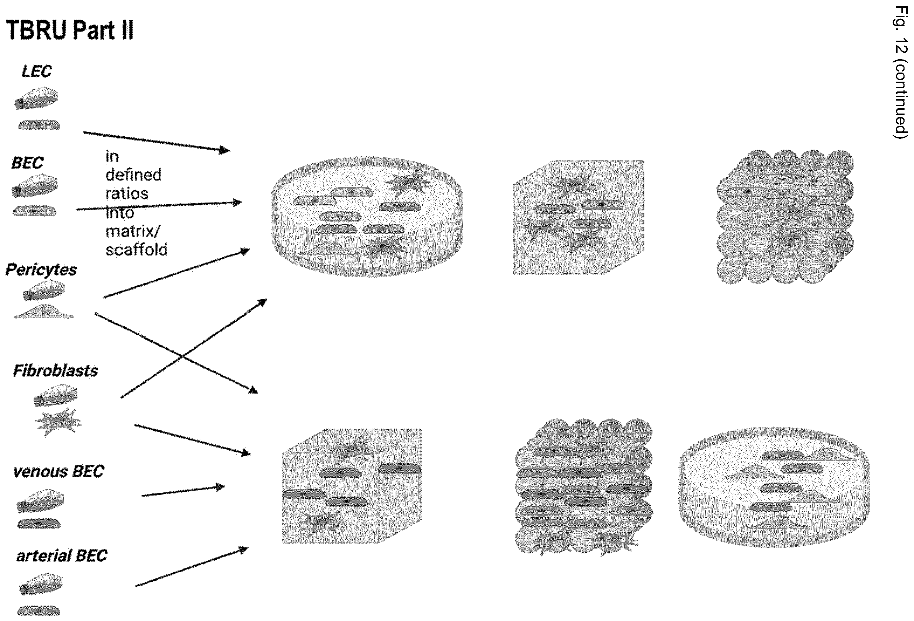

- Fig. 12: Schematic picture of the method of this application.

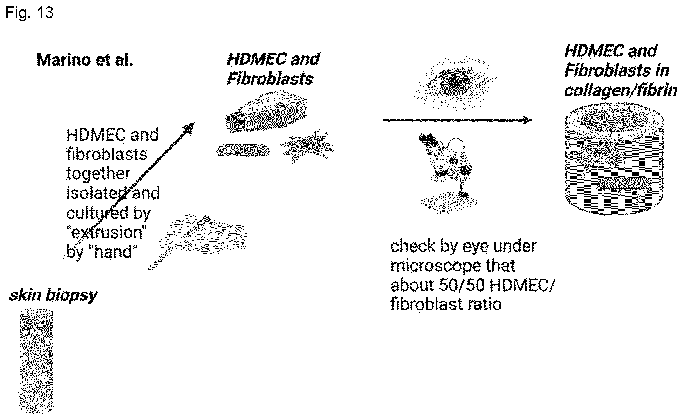

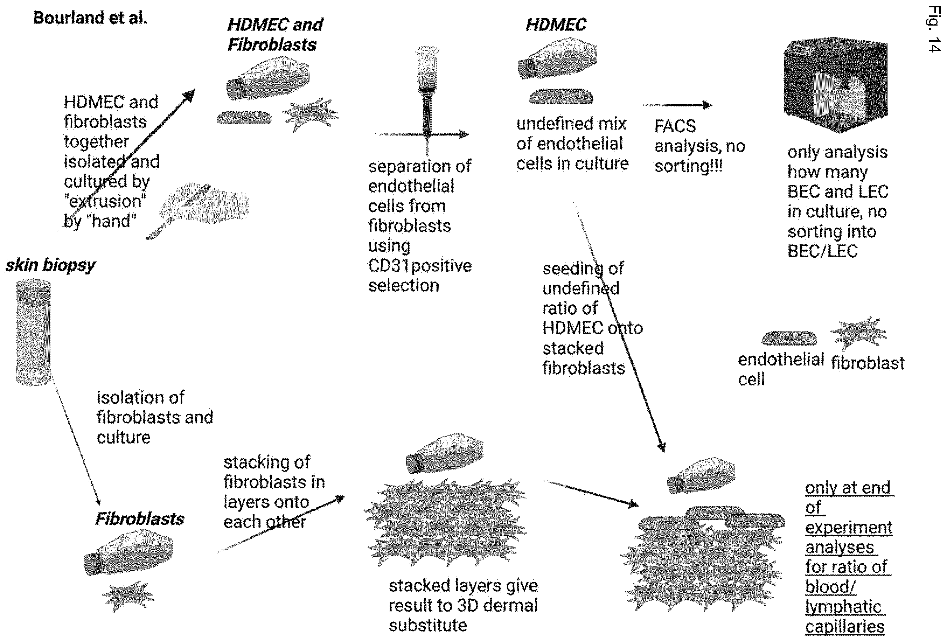

- Fig. 13: Schematic picture of the method of Marino et al. (2014) Science Translational Medicine, vol. 6, no. 221.
- Fig: 14: Schematic picture of the method of Bourland et al. (2018) Scientific Reports, vol. 8, no. 1, p. 13191.

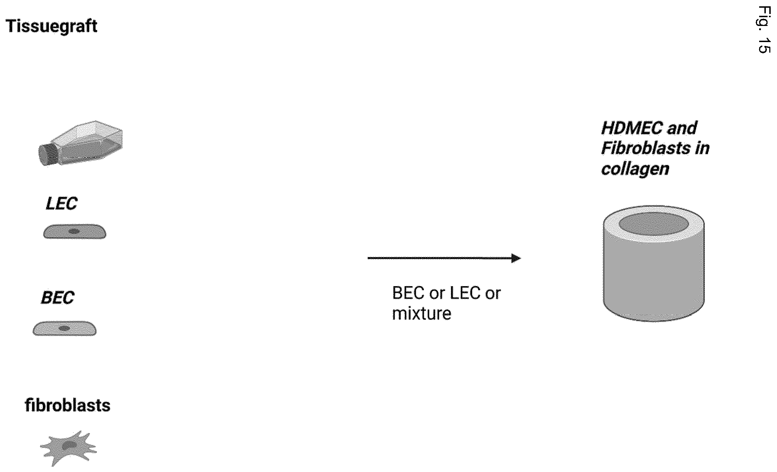

- Fig. 15: Schematic picture of the method of EP 3 174 563 B1.

### Examples

### Example 1:

Human dermal endothelial cells were isolated from a human skin biopsy and cultured on cell culture plastic at 37 °C and 5% CO₂ in appropriate endothelial cell medium (EGM2, Lonza, Switzerland). After isolation from the dermis, the cell suspension was incubated with CD31 labelled Dynabeads. CD31 positive cells were sorted from the total cell suspension and subsequently placed on a cell culture plate. The CD31 negative cells were also sorted from the total cell suspension and plated on a separate cell culture dish.

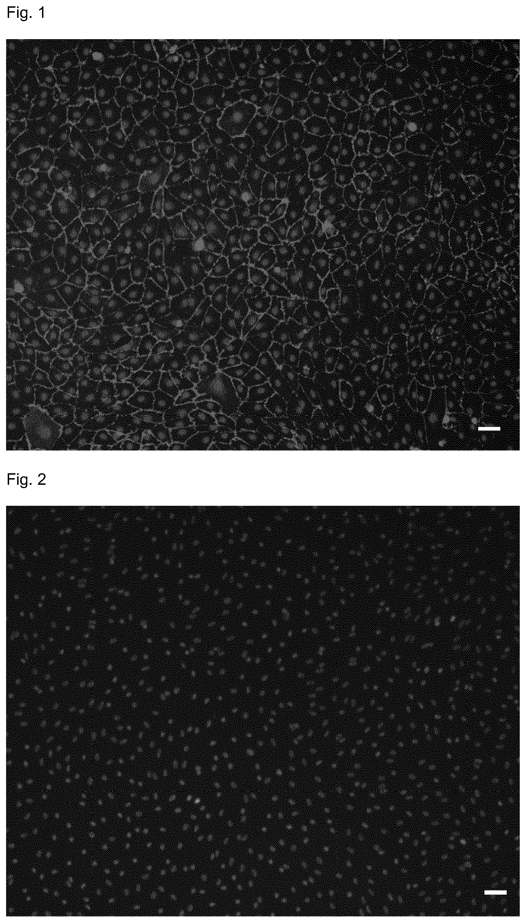

The purity of the sorted and cultured CD31 positive cells was confirmed after 5 days in culture by an immunofluorescence staining (human CD31, clone: JC70A, 1:50, Dako). The cells in culture are all positive for CD31 (Figure 1, in red), as comparison, all cell nuclei were co-stained with 1 mg/mL Hoechst 33342 (ThermoFischer, Switzerland) (Figure 1, in blue).

### Example 2:

The CD31 negative sorted cells were also analyzed by an immunofluorescence staining (human CD31, clone: JC70A, 1:50, Dako) and no expression of CD31 was observed in these cultured cells (Figure 2), as comparison all cell nuclei were stained by Hoechst 33342 (Figure 2, in blue).

### Example 3:

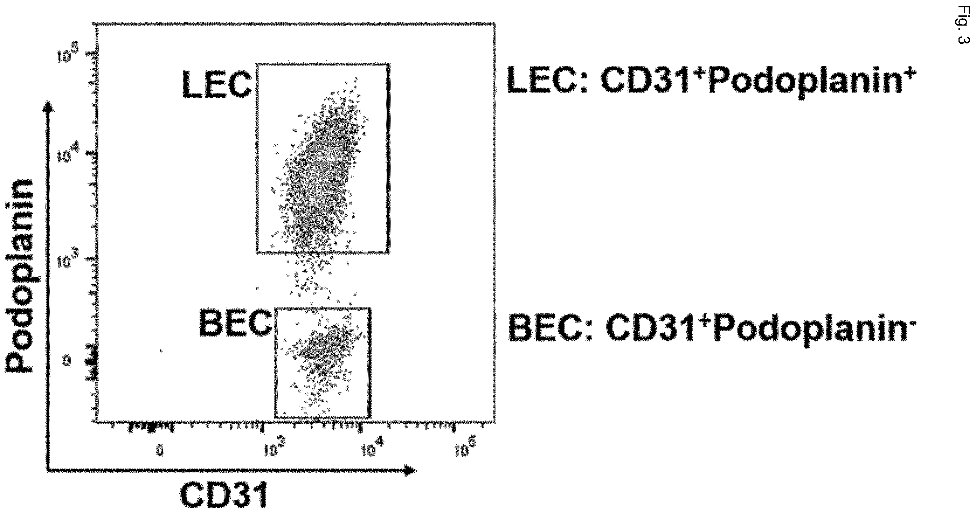

The CD31 positive sorted and cultured cells were then labelled by FACS applicable antibodies for CD31 and Podoplanin (CD31-PE, 1:20, clone WM59, BD Biosciences, Switzerland; Podoplanin-AF488clone, 1:50, NC-08, BioLegend, Switzerland) (see for method description Rütsche et al, 2022 and Michalak-Micka et al, 2022). The labelled cells were used for FACS sorting and sorted into CD31-positive/Podoplanin-positive and CD31-positive/Podoplanin-negative cells (Figure 3). Clearly, two separate cell fractions can be gated in the FACS settings and separately sorted, namely the CD31-positive/Podoplanin-positive lymphatic endothelial cell (LEC) fraction and the CD31-positive/Podoplanin-negative blood endothelial cell (BEC) fraction (Figure 3).

### Example 4:

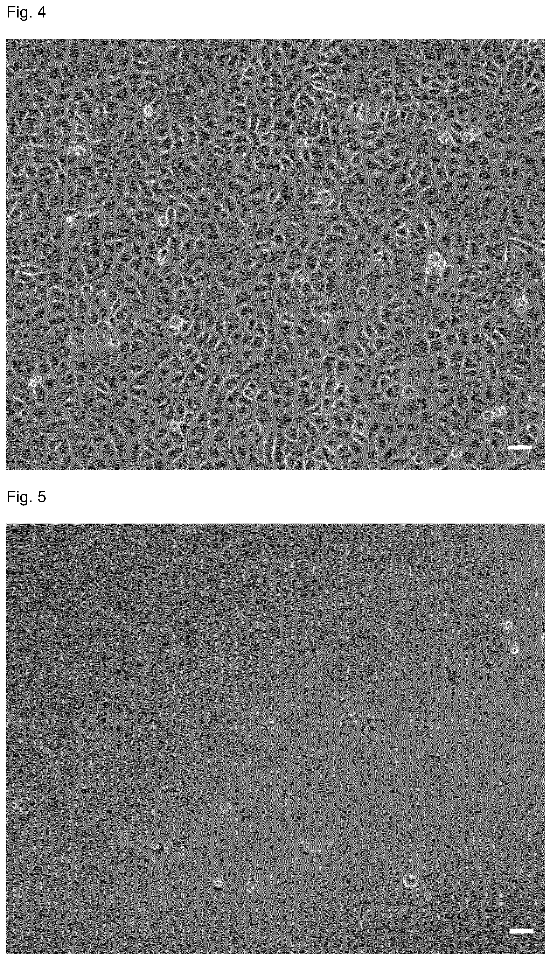

Keratinocytes and melanocytes can be isolated from the epidermis of a human skin sample as described in Michalak-Micka et al., 2022. The 2D cultured keratinocyte confluent monolayers display their typical cobblestone morphology in light microscopy (see Figure 4). Cultured melanocytes in contrast display a typical dendritic morphology in light microscopy (see Figure 5).

### Example 5:

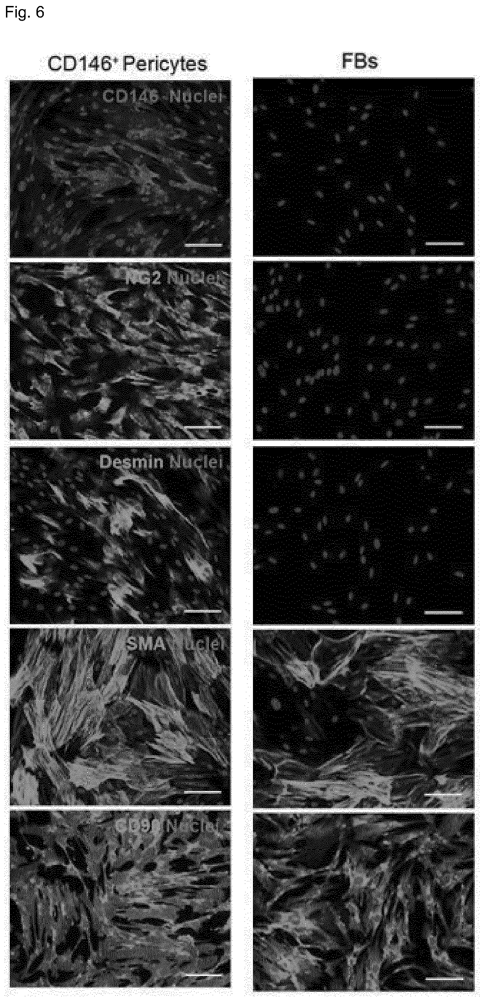

Human dermal pericytes represent a subpopulation in the total CD31-negative cell fraction isolated by Dynabeads sorting. To further separate pericytes from non-pericytes in the total CD31-negative fraction, the CD31-negative fraction was incubated with CD146 antibody (Biolegend, clone P1H12, 1:50, Switzerland) and the labelled CD146-positive pericytes were sorted (Dynabeads sorting). The CD146-positive pericytes and CD146-negative fibroblasts were further separately cultured in vitro on 2D cell culture plates (Figure 6). The difference between the fibroblasts and pericytes is demonstrated by their distinct protein expression pattern using immunofluorescence staining. Pericytes express CD146, NG2 (NG2, 1:100, clone: LHM2, Novus Biologicals, UK), Desmin (Desmin, 1:100, ab8592, Abcam, Germany), CD90 (CD90-FITC, 1:20, clone 5E10, Biolegend, Switzerland) and SMA, whereas fibroblasts do not express CD146, NG2, and Desmin, but express CD90 and SMA (SMA, 1:100, M0851, Dako, Switzerland) on cell culture plastic (Figure 6). Cell nuclei are stained by Hoechst 33342 (Figure 6, in blue).

### Example 6:

The in vitro grown prevascularized scaffolds containing human blood and lymphatic capillaries and fibroblasts were transplanted onto immune-compromised nude rats (see for description of the surgical/transplantation procedure Zimoch et al.). After 1 week in vivo, transplanted prevascularized scaffolds were excised and histological analyses were performed.

Human CD31 positive (green) endothelial cells lining human engineered capillaries were detected by immunofluorescence staining (Figure 8 A). Those human CD31 positive capillaries were present in the human scaffold delineated by human CD90 positive fibroblasts (red) to display the human tissue in comparison to host (animal) tissue (Figure 8 A). Further, human CD31 (red) positive Prox1 (green) positive lymphatic endothelial cells were demonstrated by immunofluorescence staining in comparison to CD31 positive (red) non-Prox1 (green) expressing blood endothelial cells, showing a mixture of lymphatic and blood capillaries in the human prevascularized scaffolds in vivo (Figure 8 B).

### Example 7:

### BEC-LEC containing skin substitutes show accelerated blood perfusion than non-prevascularized controls

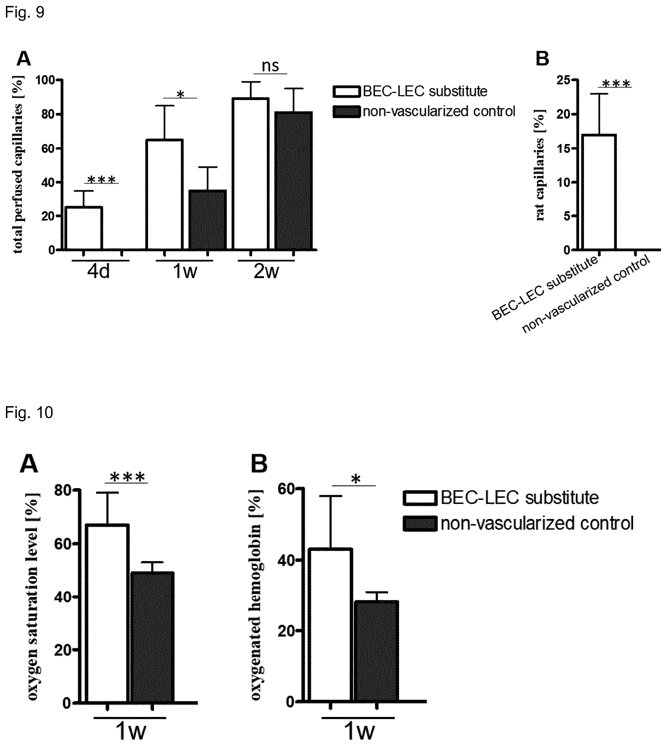

BEC-LEC skin substitutes demonstrate faster blood perfusion that non-vascularized skin substitutes, which contain only fibroblasts and lack endothelial cells (Fig. 9). As early as 4 days after transplantation, BEC-LEC skin substitutes showed 25 ± 10% human CD31⁺ capillaries connected via anastomosis to rat CD31⁺ blood capillaries (these perfused capillaries showed rat erythrocytes in their lumina), and 75 ± 12% human CD31⁺ capillaries that were not connected via anastomosis to rat CD31⁺ blood capillaries. The number of perfused capillaries increased to 65 ± 20% at 1 week, and to 89 ± 10% at 2 weeks in BEC-LEC skin substitutes of all human CD31⁺ capillaries.

Non-prevascularized skin substitutes showing 0% human CD31⁺ capillaries and thus no connection via anastomosis to rat CD31⁺ blood vessels. In addition, non-prevascularized skin substitutes showed 0% rat capillaries at day 4 in vivo, 35 ± 14% rat capillaries at 1 week, and 81 ± 14% rat capillaries at 2 weeks.

### BEC-LEC containing skin substitutes attract ingrowth of host capillaries

The inventors confirmed that BEC-LEC skin substitutes significantly accelerated the ingrowth of rat capillaries into the human dermis of the transplant. In particular, BEC-LEC skin substitutes showed presence of rat capillaries 4 days after transplantation, whereas no rat capillary was present in non-prevascularized skin substitutes 4 days after transplantation.

Importantly, BEC-LEC skin substitutes attracted the ingrowth of rat capillaries throughout the dermis up to the basement membrane (just below the epidermis) at 1 week, whereas the rat capillaries in non-prevascularized skin substitutes were identified only in the lower part of the dermis at 1 week after transplantation.

### Photo-acoustic evaluation for oxygenated blood after transplantation of BEC-LEC skin substitutes

Photo-acoustic imaging was performed to quantify in situ the oxygen saturation level and oxygenated hemoglobin content within the neo-dermis of BEC-LEC skin substitutes and controls 1 week after transplantation (Fig. 10). This approach revealed that prevascularized BEC-LEC skin substitutes were characterized by significantly higher oxygenation (67 ± 12 vs 49 ± 4 %) and oxygenated hemoglobin levels (43 ± 15 vs 28 ± 3 %) than controls at 1 week.

### Faster inflammatory response in prevascularized BEC-LEC skin substitutes after transplantation

Our data indicate that prevascularization of skin substitutes has a profound influence on the inflammatory phase during skin wound healing in vivo. In particular, the inventors observed an increased number of infiltrating monocytes/macrophages and granulocytes in prevascularized human BEC-LEC skin substitutes as compared to non-prevascularized skin substitutes after transplantation in a rat model. This is a sign of fast and pronounced wound healing in the prevascularized BEC-LEC skin substitutes. The inventors investigated in more detail the polarization state of macrophages in those prevascularized BEC-LEC skin substitutes at different wound healing stages in vivo. The inventors showed that the phenotype of macrophages changes from a pro-inflammatory M1 profile 1 week after transplantation of the prevascularized skin substitutes to an anti-inflammatory, pro-healing M2 phenotype at 3 weeks in vivo. In contrast, non-vascularized skin substitutes demonstrated in general a lower number of macrophages at 1 and 3 weeks in vivo, and in particular reduced numbers of M2 polarized macrophages. The inventors assume that the integration of a mature vascular network into skin substitutes orchestrates the fine tuning of multiple pro- and anti-inflammatory cytokines and growth factors essential at different stages of wound healing compared to non-prevascularized skin substitutes.

### Epidermal homeostasis is reached faster after transplantation of pre-vascularized skin substitutes

Epidermal keratinocytes express cytokeratins that are intermediate filaments of the cytoskeleton. After wounding, the type of cytokeratin is different than in non-wounded epidermal cells. In homeostasis, cytokeratins such as CK1 and CK10 are expressed, whereas in wound healing, cytokeratins such as CK16 and CK17 are present. After transplantation of dermo-epidermal skin substitutes that are prevascularized, CK16 and CK17 as wound healing markers are not expressed after 1-2 weeks. In contrast, in non-prevascularized skin substitutes, CK16 and CK17 are still present 14 days after transplantation and will disappear only after 3-4 weeks.

### Example 8:

The method of BEC/LEC isolation from human skin according to EP 3 174 563 B1 and Marino et al. 2014 (herein refreed to as "old method") is based on the co-culture of BEC and LEC without any separation after enzymatic extraction of HDMECs from a skin biopsy. Since the LEC demonstrate significantly higher proliferation potential in vitro, this method results in the overgrowth of BEC by LEC. Finally, the old method leads to almost pure LEC cultures after 2-3 weeks of in vitro cultures on tissue culture plastic. Hence, it is not possible to generate adequate numbers of BEC using the old method, and it is not possible to generate skin substitutes with a defined number of blood capillaries (with BEC), which are crucial for rapid oxygenation and waste removal in the skin.

### Example 9: Material and Methods

### Scaffolds

Both, size and shape depend on demands and cell culture plastic availabilities. Cell culture devices can be bought from suppliers or can be prepared custom made. Shape can for example be round (in cell culture inserts) or rectangular. As example, but not exclusively: minimal: 24 well cell culture insert size e.g., but not exclusively: maximal: 7x8 cm rectangular insert size, an example of bioprinting of a 6x6 cm size is given in Pontiggia et al., 2022.

### Mixing of cells with collagen

For a prevascularized hydrogel, 1.5 ml collagen type I (bovine collagen type I, Symatese, France) was mixed with a cell suspension of a 1:1 mixture of HDMEC and fibroblasts (100.000 cells/ml in total), casted in a 6 well insert, polymerized at 37 °C/5% CO2, after polymerization EGM-2MV medium is added and the prepared scaffold is cultured in EGM-2MV medium (Lonza, Basel, Switzerland) for 21 days to allow capillary formation at 37 °C/5% CO2. (Modified text after Zimoch et al.)

### Isolation of keratinocytes and melanocytes

Keratinocytes and melanocytes were isolated according to the description in Michalak-Micka et al., 2022. In brief, the human skin samples were first cut into small pieces and digested overnight at 4°C in a mixture of 12U/ml dispase (Corning, New York, USA) and Dulbecco's Phosphate Buffered Salin (DPBS, Sigma-Aldrich, Buchs, Switzerland) containing 5 mg/mL gentamycin (Sigma-Aldrich, Buchs, Switzerland). Thereafter, the epidermis was mechanically separated from the dermis using forceps. The epidermis was used for the isolation of keratinocytes and melanocytes. For keratinocytes isolation, the epidermis was further digested in 0.5% Trypsin-EDTA (Thermo Fisher Scientific, Basel, Switzerland) at 37°C for 2 min following cells resuspension in serum free keratinocyte medium (CnT-57, CellnTec, Bern, Switzerland) containing 5 µg/mL gentamycin. Melanocytes were harvested separately from human epidermis by the treatment with 0.05% Trypsin-EDTA at 37°C for 5 minutes. Melanocytes were cultured in melanocyte growth medium CnT40 (CellnTec, Bern, Switzerland). Medium was changed every second day.

### Cited prior art documents:

Zimoch et al., Acta Biomaterialia, Volume 134, 15 October 2021, Pages 215-227.
Pontiggia et al., J Tissue Eng. 2022 Apr 25; 13:20417314221088513.
Michalak-Micka et al., Cell Reports, Volume 38, Issue 9, 1 March 2022, 110419.
Rütsche et al., 2022, Cells 2022, 11(6), 1055.
Marino et al. (2014) Science Translational Medicine, vol. 6, no. 221
Bourland et al. (2018) Scientific Reports, vol. 8, no. 1, p. 13191
EP 3 174 563 B1

## Claims

1. A method for production of a skin substitute, said method comprising the steps:
a. providing a skin tissue sample isolated from a mammalian subject;
b. separating the skin tissue sample into dermis and epidermis;
c. enzymatically degrading matrix components of the dermis to obtain a plurality of cells in suspension;
d. in a CD31 step, sorting said plurality of cells into a CD31⁺ cell fraction and a CD31⁻ cell fraction;
e. culturing the CD31⁺ cell fraction yielding a plurality of CD31⁺ cells;
f. in a BEC/LEC step, sorting said plurality of CD31⁺ cells into blood endothelial cells (BEC), and lymphatic endothelial cells (LEC) using a BEC-specific and/or LEC-specific marker;
g. in a culturing step, culturing BEC and/or LEC yielding a skin substitute.

2. The method according to claim 1, wherein in the culturing step, a relative amount of BEC to LEC is used between 70% BEC and 30% LEC to 30% BEC and 70% LEC.

3. The method according to any one of the preceding claims, wherein the CD31 step is performed via magnetic sorting, particularly wherein the CD31 step is performed via paramagnetic particles.

4. The method according to any one of the preceding claims, wherein the BEC/LEC step is performed via fluorescence-based sorting or magnetic sorting, particularly via FACS.

5. The method according to any one of the preceding claims, wherein in the BEC/LEC step,
the BEC-specific marker is selected from the group consisting of PLVAP, PAL-E, and/or
the LEC-specific marker is selected from the group consisting of podoplanin, Lyve1, VEGFR3.

6. The method according to any one of the preceding claims, wherein after the BEC/LEC step, BEC are sorted into arterial and venous endothelial cells via an arterial endothelial cell- and/or a venous endothelial cell-specific marker, wherein the arterial endothelial cell-specific marker is selected from the group consisting of Ephrin B2 positive, Neuropilin 1 (NRP1)^{high}, and wherein the venous endothelial cell-specific marker is selected from the group consisting of ACKR1 (DARC) positive, Neuropilin 2 (NRP2)^{high}.

7. The method according to any one of the preceding claims, wherein during the BEC/LEC step, additionally CD146⁺ mural cells are sorted from the CD31⁻ cell fraction and are added in the culturing step.

8. The method according to any one of the preceding claims, wherein keratinocytes are isolated from the epidermis
- via treatment of the epidermis with ~0.5% (w/v) trypsin solution, or
- via sorting cells of the epidermis into a MC1R negative/E-cadherin positive fraction;
and keratinocytes are added in the culturing step.

9. The method according to any one of the preceding claims, wherein melanocytes are isolated from the epidermis
- via treatment with ~0.05% (w/v) trypsin solution, or
- via sorting cells of the epidermis into a MC1R positive/E-cadherin positive fraction;
and melanocytes are added in the culturing step.

10. The method according to any one of the preceding claims, wherein digestion of the tissue sample is performed using an enzyme or an enzyme combination selected from the group consisting of collagenase II, and/or collagenase IV, and/or a mixture of collagenase I and collagenase II, and/or trypsin.

11. The method according to any one of the preceding claims, wherein in the culturing step, additionally
- collagen, and/or
- fibrin, and/or
- gelatin and/or
- PEG and/or
- polyurethane
is added.

12. A skin substitute obtained by the method according to any one of the preceding claims, wherein a dermal part of the skin substitute comprises ≥35% endothelial cells.

13. The skin substitute according to claim 12, wherein the skin substitute, if transplanted to a recipient subject,
- is capable of being connected to recipient subject's CD31⁺ blood capillaries 4 days after transplantation to the recipient subject, particularly wherein >15% of donor CD31⁺ blood capillaries of the skin substitute are capable of being connected to recipient subject's CD31⁺ blood capillaries 4 days after transplantation to the recipient subject; and/or
- is capable of attracting ingrowth of recipient subject's blood capillaries throughout the dermis up to the basement membrane, particularly the skin substitute, if transplanted to a recipient subject, is capable of attracting ingrowth of recipient subject's blood capillaries throughout the dermis up to the basement membrane at one week after transplantation to the recipient subject; and/or
- is capable of attracting ingrowth of recipient subject's blood capillaries at 4 days after transplantation to the recipient subject; and/or
- is capable of showing an oxygen saturation level of >50% at one week after transplantation to the recipient subject; and/or
- is capable of having oxygenated hemoglobin levels of >30% at one week after transplantation to the recipient subject; and/or
- is capable of comprising a higher number of M2 macrophages than number of M1 macrophages at three weeks after transplantation to the recipient subject; and/or
- is **characterized by** low or absent expression of CK16 and CK17 at two weeks after transplantation to the recipient subject, particularly at one week after transplantation to the recipient subject.

14. The skin substitute according to claim 12 or 13, wherein all cells of the skin substitute are derived from a single donor subject, particularly wherein the donor subject is the recipient (autologous transplantation).

15. A skin substitute according to claim 12 to 14 for use in treatment of a condition selected from the group consisting of:
- a secondary lymphoedema, particularly wherein the skin substitute was cultured from a relative BEC/LEC amount of 0% BEC and 100% LEC;
- a chronic edema, particularly wherein the skin substitute was cultured from a relative BEC/LEC amount of ~50% BEC and ~50% LEC;
- an acute burn wound, particularly wherein the skin substitute was cultured from a relative BEC/LEC amount of 100% BEC and 0% LEC;
- an arterial ulcer, particularly wherein the skin substitute was cultured from a relative BEC/LEC amount of 100% BEC and 0% LEC;
- a venous leg ulcer, particularly wherein the skin substitute was cultured from a relative BEC/LEC amount of 100% BEC and 0% LEC;
- lymphatic filariasis, particularly wherein the skin substitute was cultured from a relative BEC/LEC amount of 0% BEC and 100% LEC;
- phlebolymphedema, particularly wherein the skin substitute was cultured from a relative BEC/LEC amount of ~50% BEC and ~50% LEC;
- lymphangioleiomyomatosis, particularly wherein the skin substitute was cultured from a relative BEC/LEC amount of 0% BEC and 100% LEC;
- skin defect, wherein the skin substitute was cultured from a relative BEC/LEC amount as present in the defected skin.

## Patentansprüche

1. Ein Verfahren zur Herstellung eines Hautersatzes, wobei das Verfahren die folgenden Schritte umfasst:
- bereitstellen einer aus einem Säugetier isolierten Hautgewebeprobe;
- trennen der Hautgewebeprobe in Dermis und Epidermis;
- enzymatisches abbauen von Matrixkomponenten der Dermis, um eine Vielzahl von Zellen in Suspension zu erhalten;
- in einem CD31-Schritt, sortieren der Vielzahl von Zellen in eine CD31+-Zellfraktion und eine CD31-Zellfraktion;
- kultivieren der CD31+-Zellfraktion, wodurch eine Vielzahl von CD31+-Zellen gewonnen wird;
- in einem BEC/LEC-Schritt sortieren der Vielzahl von CD31+-Zellen in Blutendothelzellen (BEC) und Lymphendothelzellen (LEC) unter Verwendung eines BEC-spezifischen und/oder LEC-spezifischen Markers;
- in einem Kultivierungsschritt kultivieren von BEC und/oder LEC, wodurch ein Hautersatz gewonnen wird.

2. Das Verfahren nach Anspruch 1, wobei im Kultivierungsschritt ein relativer Anteil von BEC zu LEC verwendet wird, der zwischen 70 % BEC und 30 % LEC, und 30 % BEC und 70 % LEC liegt.

3. Das Verfahren nach einem der vorstehenden Ansprüche, wobei der CD31-Schritt mittels magnetischer Sortierung durchgeführt wird, insbesondere wobei der CD31-Schritt mittels paramagnetischer Partikel durchgeführt wird.

4. Das Verfahren nach einem der vorstehenden Ansprüche, wobei der BEC/LEC-Schritt mittels fluoreszenzbasierter Sortierung oder magnetischer Sortierung durchgeführt wird, insbesondere mittels FACS.

5. Das Verfahren nach einem der vorstehenden Ansprüche, wobei im BEC/LEC-Schritt
der BEC-spezifische Marker aus der Gruppe bestehend aus PLVAP, PAL-E ausgewählt wird,
und/oder
der LEC-spezifische Marker aus der Gruppe bestehend aus Podoplanin, Lyve1 und VEGFR3 ausgewählt wird.

6. Das Verfahren nach einem der vorstehenden Ansprüche, wobei nach dem BEC/LEC-Schritt, BEC anhand eines für arterielle Endothelzellen und/oder eines für venöse Endothelzellen spezifischen Markers in arterielle und venöse Endothelzellen sortiert werden, wobei der für arterielle Endothelzellen spezifische Marker aus der Gruppe bestehend aus Ephrin B2-positiv, Neuropilin 1 (NRP1)high ausgewählt wird, und wobei der venöse endothelzellspezifische Marker aus der Gruppe bestehend aus ACKR1 (DARC)-positiv und Neuropilin 2 (NRP2)high ausgewählt wird.

7. Das Verfahren nach einem der vorstehenden Ansprüche, wobei während des BEC/LEC-Schritts zusätzlich CD146+-Muralzellen aus der CD31--Zellfraktion sortiert und im Kultivierungsschritt zugegeben werden.

8. Das Verfahren nach einem der vorstehenden Ansprüche, wobei Keratinozyten aus der Epidermis isoliert werden
- durch Behandlung der Epidermis mit einer ~0,5 % (w/v) Trypsinlösung, oder
- durch sortieren der Zellen der Epidermis in eine MC1R-negative/E-Cadherinpositive Fraktion;
und Keratinozyten im Kultivierungsschritt zugegeben werden.

9. Das Verfahren nach einem der vorstehenden Ansprüche, wobei Melanozyten aus der Epidermis isoliert werden
- durch Behandlung mit einer ~0,5 % (w/v) Trypsinlösung, oder
- durch sortieren der Zellen der Epidermins in eine MC1R-positive/E-Cadherinpositive Fraktion;
und die Melanozyten im Kultivierungsschritt zugegeben werden.

10. Das Verfahren nach einem der vorstehenden Ansprüche, wobei die Aufspaltung der Gewebeprobe unter Verwendung eines Enzyms oder einer Enzymkombination durchgeführt wird, die aus der Gruppe bestehend aus Kollagenase II und/oder Kollagenase IV und/oder einer Mischung aus Kollagenase I und Kollagenase II und/oder Trypsin ausgewählt ist.

11. Das Verfahren nach einem der vorstehenden Ansprüche, wobei im Kultivierungsschritt zusätzlich
- Kollagen und/oder
- Fibrin und/oder
- Gelatine und/oder
- PEG und/oder
- Polyurethan
zugesetzt wird.

12. Ein Hautersatz, der nach dem Verfahren gemäß einem der vorstehenden Ansprüche hergestellt wurde, wobei ein dermaler Teil des Hautersatzes ≥ 35 % Endothelzellen umfasst.

13. Der Hautersatz nach Anspruch 12, wobei der Hautersatz, wenn er einem Empfänger transplantiert wird,
- 4 Tage nach der Transplantation in einen Empfänger eine Verbindung zu den CD31+-Blutkapillaren des Empfängers herstellen kann, wobei insbesondere >15 % der Spender-CD31+-Blutkapillaren des Hautersatzes 4 Tage nach der Transplantation in den Empfänger eine Verbindung zu den CD31+-Blutkapillaren des Empfängers herstellen können; und/oder
- 4 Tage nach der Transplantation in den Empfänger das Einwachsen von Blutkapillaren des Empfängers fördern kann; und/oder
- eine Woche nach der Transplantation in den Empfänger eine Sauerstoffsättigung von >50 % aufweisen kann; und/oder
- eine Woche nach der Transplantation in den Empfänger einen Gehalt an sauerstoffgesättigtem Hämoglobin von >30 % aufweisen kann; und/oder
- drei Wochen nach der Transplantation in den Empfänger eine höhere Anzahl an M2-Makrophagen als an M1-Makrophagen aufweisen kann; und/oder
- durch eine geringe oder fehlende Expression von CK16 und CK17 zwei Wochen nach der Transplantation in den Empfänger, insbesondere eine Woche nach der Transplantation in den Empfänger, gekennzeichnet ist.

14. Der Hautersatz gemäß Anspruch 12 oder 13, wobei alle Zellen des Hautersatzes von einem einzigen Spender stammen, insbesondere wobei der Spender der Empfänger ist (autologe Transplantation).

15. Ein Hautersatz gemäß den Ansprüchen 12 bis 14 zur Verwendung bei der Behandlung eines Zustands, ausgewählt aus der Gruppe bestehend aus:
- einem sekundären Lymphödem, insbesondere wobei der Hautersatz aus einem relativen BEC/LEC-Anteil von 0 % BEC und 100 % LEC kultiviert wurde;
- ein chronisches Ödem, insbesondere wobei der Hautersatz aus einem relativen BEC/LEC-Verhältnis von ~50 % BEC und ~50 % LEC kultiviert wurde;
- eine akute Verbrennungswunde, insbesondere wobei der Hautersatz aus einem relativen BEC/LEC-Verhältnis von 100 % BEC und 0 % LEC kultiviert wurde;
- ein arterielles Geschwür, insbesondere wobei der Hautersatz aus einem relativen BEC/LEC-Verhältnis von 100 % BEC und 0 % LEC kultiviert wurde;
- ein venöses Beingeschwür, insbesondere wobei der Hautersatz aus einem relativen BEC/LEC-Verhältnis von 100 % BEC und 0 % LEC kultiviert wurde;
- lymphatische Filariose, insbesondere wobei der Hautersatz aus einem relativen BEC/LEC-Verhältnis von 0 % BEC und 100 % LEC kultiviert wurde;
- Phlebolymphödem, insbesondere wobei der Hautersatz aus einem relativen BEC/LEC-Verhältnis von ~50 % BEC und ~50 % LEC kultiviert wurde;
- Lymphangioleiomyomatose, insbesondere wobei der Hautersatz aus einem relativen BEC/LEC-Verhältnis von 0 % BEC und 100 % LEC kultiviert wurde;
- Hautdefekt, wobei der Hautersatz aus einem relativen BEC/LEC-Verhältnis kultiviert wurde, wie es in der defekten Haut vorliegt.

## Revendications

1. Procédé de production d'un substitut cutané, ledit procédé comprenant les étapes :
a. fournir un échantillon de tissu cutané isolé d'un sujet mammifère ;
b. séparer l'échantillon de tissu cutané en derme et épiderme ;
c. dégrader par voie enzymatique des composants de matrice du derme pour obtenir une pluralité de cellules en suspension ;
d. dans une étape CD31, trier ladite pluralité de cellules en une fraction de cellules CD31⁺ et une fraction de cellules CD31⁻ ;
e. mettre en culture la fraction de cellules CD31⁺ donnant une pluralité de cellules CD31⁺ ;
f. dans une étape BEC/LEC, trier ladite pluralité de cellules CD31⁺ en des cellules endothéliales sanguines (BEC) et des cellules endothéliales lymphatiques (LEC) en utilisant un marqueur spécifique de BEC et/ou spécifique de LEC ;
g. dans une étape de mise en culture, mettre en culture des BEC et/ou LEC donnant un substitut cutané.

2. Procédé selon la revendication 1, dans lequel dans l'étape de mise en culture, une quantité relative de BEC sur LEC est utilisée entre 70 % de BEC et 30 % de LEC sur 30 % de BEC et 70 % de LEC.

3. Procédé selon l'une quelconque des revendications précédentes, dans lequel l'étape CD31 est réalisée par le biais d'un tri magnétique, notamment dans lequel l'étape CD31 est réalisée par le biais de particules paramagnétiques.

4. Procédé selon l'une quelconque des revendications précédentes, dans lequel l'étape BEC/LEC est réalisée par le biais d'un tri à base de fluorescence ou d'un tri magnétique, notamment par le biais d'un trieur de cellules à fluorescence FACS.

5. Procédé selon l'une quelconque des revendications précédentes, dans lequel dans l'étape BEC/LEC,
le marqueur spécifique de BEC est sélectionné parmi le groupe constitué de PLVAP, PAL-E et/ou
le marqueur spécifique de LEC est sélectionné parmi le groupe constitué de la podoplanine, Lyve1, VEGFR3.

6. Procédé selon l'une quelconque des revendications précédentes, dans lequel après l'étape BEC/LEC, les BEC sont triées en cellules endothéliales artérielles et veineuses par le biais d'un marqueur spécifique de cellule endothéliale artérielle et/ou de cellule endothéliale veineuse, dans lequel le marqueur spécifique de cellule endothéliale artérielle est sélectionné parmi le groupe constitué de l'éphrine B2 positive, la neuropiline 1 (NRP1)^{high}, et dans lequel le marqueur spécifique de cellule endothéliale veineuse est sélectionné parmi le groupe constitué de ACKR1 (DARC) positif, la neuropiline 2 (NRP2)^{high}.

7. Procédé selon l'une quelconque des revendications précédentes, dans lequel au cours de l'étape BEC/LEC, des cellules murales CD146⁺ sont en outre triées à partir de la fraction de cellules CD31⁻ et sont ajoutées dans l'étape de mise en culture.

8. Procédé selon l'une quelconque des revendications précédentes, dans lequel des kératinocytes sont isolés de l'épiderme
- par le biais d'un traitement de l'épiderme avec ~0,5 % (p/v) de solution de trypsine, ou
- par le biais d'un tri de cellules de l'épiderme en une fraction négative à MC1R/positive à l'E-cadhérine ;
et des kératinocytes sont ajoutés dans l'étape de mise en culture.

9. Procédé selon l'une quelconque des revendications précédentes, dans lequel des mélanocytes sont isolés de l'épiderme
- par le biais d'un traitement avec ~0,05 % (p/v) de solution de trypsine, ou
- par le biais d'un tri de cellules de l'épiderme en une fraction positive à MC1R/positive à l'E-cadhérine ;
et des mélanocytes sont ajoutés dans l'étape de mise en culture.

10. Procédé selon l'une quelconque des revendications précédentes, dans lequel la digestion de l'échantillon de tissu est réalisée en utilisant une enzyme ou une combinaison d'enzymes sélectionnées parmi le groupe constitué de la collagénase II et/ou collagénase IV, et/ou d'un mélange de collagénase I et collagénase II, et/ou de la trypsine.

11. Procédé selon l'une quelconque des revendications précédentes, dans lequel dans l'étape de mise en culture,
- du collagène, et/ou
- de la fibrine, et/ou
- de la gélatine et/ou
- du PEG et/ou
- du polyuréthane
est en outre ajouté(e).

12. Substitut cutané obtenu par le procédé selon l'une quelconque des revendications précédentes, dans lequel une partie dermique du substitut cutané comprend ≥35 % de cellules endothéliales.

13. Substitut cutané selon la revendication 12, dans lequel le substitut cutané, s'il est greffé vers un sujet receveur,
- est capable d'être connecté aux capillaires sanguins CD31⁺ du sujet receveur 4 jours après la greffe vers le sujet receveur, notamment dans lequel >15 % de capillaires sanguins CD31⁺ donneurs du substitut cutané sont capables d'être connectés aux capillaires sanguins CD31⁺ du sujet receveur 4 jours après la greffe vers le sujet receveur ; et/ou
- est capable d'attirer l'enracinement de capillaires sanguins du sujet receveur à travers tout le derme jusqu'à la membrane basale, notamment le substitut cutané, s'il est greffé vers un sujet receveur, est capable d'attirer l'enracinement de capillaires sanguins du sujet receveur à travers tout le derme jusqu'à la membrane basale à une semaine après la greffe vers le sujet receveur ; et/ou
- est capable d'attirer l'enracinement de capillaires sanguins du sujet receveur à 4 jours après la greffe vers le sujet receveur ; et/ou
- est capable de présenter un niveau de saturation en oxygène de >50 % à une semaine après la greffe vers le sujet receveur ; et/ou
- est capable d'avoir des niveaux d'hémoglobine oxygénée de >30 % à une semaine après la greffe vers le sujet receveur ; et/ou
- est capable de comprendre un nombre supérieur de macrophages M2 au nombre de macrophages M1 à trois semaines après la greffe vers le sujet receveur ; et/ou
- est **caractérisé par** une faible expression ou expression absente de CK16 et CK17 à deux semaines après la greffe vers le sujet receveur, notamment à une semaine après la greffe vers le sujet receveur.

14. Substitut cutané selon la revendication 12 ou 13, dans lequel l'ensemble des cellules du substitut cutané sont dérivées d'un seul sujet donneur, notamment dans lequel le sujet donneur est le receveur (greffe autologue).

15. Substitut cutané selon les revendications 12 à 14 destiné à être utilisé dans le traitement d'une affection sélectionnée parmi le groupe constitué de :
- un lymphœdème secondaire, notamment dans lequel le substitut cutané a été mis en culture à partir d'une quantité de BEC/LEC relative de 0 % de BEC et 100 % de LEC ;
- un œdème chronique, notamment dans lequel le substitut cutané a été mis en culture à partir d'une quantité de BEC/LEC relative de ~50 % de BEC et ~50 % de LEC ;
- une plaie de brûlure aiguë, notamment dans lequel le substitut cutané a été mis en culture à partir d'une quantité de BEC/LEC relative de 100 % de BEC et 0 % de LEC ;
- un ulcère artériel, notamment dans lequel le substitut cutané a été mis en culture à partir d'une quantité de BEC/LEC relative de 100 % de BEC et 0 % de LEC ;
- un ulcère veineux de la jambe, notamment dans lequel le substitut cutané a été mis en culture à partir d'une quantité de BEC/LEC relative de 100 % de BEC et 0 % de LEC ;
- la filariose lymphatique, notamment dans lequel le substitut cutané a été mis en culture à partir d'une quantité de BEC/LEC relative de 0 % de BEC et 100 % de LEC ;
- un phlébolymphœdème, notamment dans lequel le substitut cutané a été mis en culture à partir d'une quantité de BEC/LEC relative de ~50 % de BEC et ~50 % de LEC ;
- la lymphangioléiomyomatose, notamment dans lequel le substitut cutané a été mis en culture à partir d'une quantité de BEC/LEC relative de 0 % de BEC et 100 % de LEC ;
- un défaut cutané, dans lequel le substitut cutané a été mis en culture à partir d'une quantité de BEC/LEC relative telle que présente dans le défaut cutané.
